# EUROPEAN PATENT SPECIFICATION

(11) **EP 4 610 271 B1**
(45) Date of publication and mention of the grant of the patent: **02.09.2026**
(21) Application number: 23926950.9
(22) Date of filing: 07.06.2023
(51) Int. Cl.: C07K 7/08, A61L 24/10, A61L 24/00, C12N 5/00

(54) **SELF-ASSEMBLING POLYPEPTIDE AND USE THEREOF**
SELBSTANORDNENDES POLYPEPTID UND VERWENDUNG DAVON
POLYPEPTIDE AUTO-ASSEMBLÉ ET SON UTILISATION

(30) Priority: 16.03.2023 CN 202310273940
(43) Date of publication of application: 03.09.2025
(73) Proprietor: Success Bio-Tech Co., Ltd., Jinan, Shandong 250101 (CN)
(72) Inventor: ZOU, Fangming, Jinan, Shandong 250101 (CN); ZHANG, Shang, Jinan, Shandong 250101 (CN); WANG, Pin, Jinan, Shandong 250101 (CN); CHENG, Yu, Jinan, Shandong 250101 (CN); GAO, Lichang, Jinan, Shandong 250101 (CN); LI, Gang, Jinan, Shandong 250101 (CN); LI, Fangmin, Jinan, Shandong 250101 (CN); LIU, Yuanxue, Jinan, Shandong 250101 (CN); ZHU, Jinfeng, Jinan, Shandong 250101 (CN); ZHANG, Linlin, Jinan, Shandong 250101 (CN); ZHANG, Chunxia, Jinan, Shandong 250101 (CN); ZHAO, Chengru, Jinan, Shandong 250101 (CN); XIA, Yiran, Jinan, Shandong 250101 (CN); BAI, Huan, Jinan, Shandong 250101 (CN)
(74) Representative: Lorenz Seidler Gossel Part. mbB
(86) International application number: PCT/CN2023/098780
(87) International publication number: WO 2024/187601

(56) References cited:
- WO-A1-2022/028036
- WO-A1-2022/028036
- CN-A- 111 848 736
- CN-A- 113 234 125
- CN-A- 114 805 484
- CN-A- 116 178 498
- FANGMIN ET AL: ""PP-type" self-assembling peptides with superior rheological properties - PMC", vol. 3, no. 21, 1 January 2021 (2021-01-01), pages 6056 - 6062, XP093356104, ISSN: 2516-0230, Retrieved from the Internet <URL:https://pmc.ncbi.nlm.nih.gov/articles/PMC9417066/> DOI: 10.1039/D1NA00534K
- DING XIN ET AL: "Synthetic peptide hydrogels as 3D scaffolds for tissue engineering", ADVANCED DRUG DELIVERY REVIEWS, ELSEVIER, AMSTERDAM , NL, vol. 160, 1 January 2020 (2020-01-01), pages 78 - 104, XP086398254, ISSN: 0169-409X, [retrieved on 20201019], DOI: 10.1016/J.ADDR.2020.10.005
- ZOU PENGFEI ET AL: "Recent advances: peptides and self-assembled peptide-nanosystems for antimicrobial therapy and diagnosis", vol. 8, no. 18, 1 January 2020 (2020-01-01), pages 4975 - 4996, XP093209692, ISSN: 2047-4830, Retrieved from the Internet <URL:https://pubs.rsc.org/en/content/articlepdf/2020/bm/d0bm00789g> DOI: 10.1039/D0BM00789G
- LI HONG, XIAODAN SHI, JIELING LI: "Self-Assembled Peptide Hydrogel for Biomedical Applications ", PROGRESS IN CHEMISTRY, vol. 34, no. 3, 1 January 2022 (2022-01-01), pages 568 - 579, XP093171933, DOI: 10.7536/PC210613
- ZOU PENGFEI, CHEN WEN-TING, SUN TONGYI, GAO YUANYUAN, LI LI-LI, WANG HAO: "Recent advances: peptides and self-assembled peptide-nanosystems for antimicrobial therapy and diagnosis", BIOMATERIALS SCIENCE, R S C PUBLICATIONS, GB, vol. 8, no. 18, 15 September 2020 (2020-09-15), GB , pages 4975 - 4996, XP093209692, ISSN: 2047-4830, DOI: 10.1039/D0BM00789G

## Description

The present invention claims the priority of Chinese patent application No. 202310273940.7, filed with the China Patent Office on March 16, 2023, entitled "Self-assembling peptides and applications thereof".

### TECHNICAL FIELD

The present invention belongs to the field of biomedical technology and relates to hydrogels for medical uses, in particular to a self-assembling peptide and applications thereof.

### BACKGROUND

The disclosure of this background section is merely intended to increase the understanding of the overall background of the present invention, and is not necessarily considered an admission or in any form to imply that the information constitutes prior art known to those of ordinary skill in the art.

Ionic-complementary self-assembling peptides (ISAPs) are biomaterials composed of peptides, whose gelation mechanism is physical self-assembly, referred to as ISAPs or the singular form ISAP or peptide in the following text. ISAPs have special amino acid sequence patterns, usually consisting of repeating positively charged amino acids, hydrophobic amino acids, negatively charged amino acids, and hydrophobic amino acids. For example, the peptide composed of the classic sequence AC-RADARADARADARADA-NH₂ (RADA16). ISAPs and their mechanical strength are sequence-dependent, using amino acids which are more hydrophobic can increase the mechanical strength of self-assembling peptide gels, but it also brings problems such as difficulty in synthesis, difficulty in purification, insolubility, and the increased viscosity of the peptide solution. In previous research of the present invention, a PP-type self-assembling peptide was provided, which through adding proline P at both ends of the self-assembling peptide, not only increased the liquid-solid phase transition capability of the self-assembling peptide but also reduced the difficulty of synthesis and purification and reduced the viscosity of the peptide solution. The PP-type self-assembling peptide is such as AC-PRVDVRVDP-NH₂ (PRVDP9).

Generally, when using self-assembling peptides as hemostatic, sealing or embolizing hydrogels for medical uses, or as cell culture scaffolds, they need to remain in solution state prior to triggering by triggering factors in order to enable delivery via catheters to the wound site or to facilitate the mixing with cells. However, the inventor's study found that both PRVDP9 and RADA16 need to be under low pH conditions (typically pH < 3) to stay in solution. If the pH of the ISAP solution increases, the solution will turn into gel, or the peptide will precipitate. However, when used as cell culture scaffolds, if the pH of the solution is too low, it will be unfavorable for cell culture. Application scenarios of self-assembling peptides as hemostatic, sealing or embolizing hydrogels for medical uses include closure of surgical wounds, hemostasis, closure of pulmonary air leaks, closure of the dura mater, closure of anastomosis surgery and intravascular interventional embolization etc., which need to be performed *in vivo,* but the pH of body fluids *in vivo* is relatively stable. Therefore, when used *in vivo,* if the pH of the self-assembling peptide is too low, the acidity will be difficult to be neutralized, which will affect the pH of body fluids *in vivo* and be unfavorable for *in vivo* application.

Documents relevant to the technical background include the following.

Document D1 (Li et al., "PP-type self-assembling peptides with superior rheological properties") describes PP-type self-assembling peptides and their rheological properties.

Document D2 (Ding et al., "Synthetic peptide hydrogels as 3D scaffolds for tissue engineering") reviews synthetic peptide hydrogels used as three-dimensional scaffolds for tissue engineering.

Document D3 (WO 2022/028036 A1) discloses, inter alia, a self-assembling peptide having the sequence AC-Pro-Arg-Val-Asp-Val-Arg-Val-Asp-Pro-amide, as well as preparations comprising the peptide in powder or liquid form.

Document D4 (Zou et al., "Recent advances: peptides and self-assembled peptide-nanosystems for antimicrobial therapy and diagnosis") reviews peptides and self-assembled peptide nanosystems for antimicrobial therapy and diagnosis.

### SUMMARY

The purpose of the present invention is to provide a self-assembling peptide and applications thereof, which overcome the shortcomings of the existing technology. The solution of the self-assembling peptides not only can maintain a solution state under conditions of higher pH, but also the acidity of the solution is easier to be neutralized, thereby avoiding affecting the pH of body fluids *in vivo.*

To achieve the objective, the present invention relates to a self-assembling peptide whose amino acid sequence consists of the amino acid sequence set forth in SEQ ID NO: 1.

First, through research on several peptides, it was found that the aforementioned self-assembling peptide can easily dissolve in water and form good gels when mixed with Dulbecco's Modified Eagle's Medium (DMEM) culture medium, Phosphate Buffer Solution (PBS), or body fluids such as blood, etc.

Secondly, the solution of the self-assembling peptide has a liquid-solid phase change pH condition of around 6 under the concentration of 1% w/v (g/mL, the same below), which is closer to the pH of body fluids.

Thirdly, it was unexpectedly found in the study that cells can maintain good proliferation on the gel formed by the aforementioned self-assembling peptide, indicating that the self-assembling peptide has good cytocompatibility and biocompatibility.

In addition, it was found through experiments that, compared with the PP-type self-assembling peptides and the classic RADA16 from the previous studies, the gel formed by the aforementioned self-assembling peptide has better mechanical properties.

The self-assembling peptide may be formulated as a peptide composition comprising the self-assembling peptide described above and at least a pharmaceutical excipient.

The self-assembling peptide may also be provided as a peptide preparation, wherein the peptide preparation is in a form of a powder or a solution.

The self-assembling peptide, the peptide composition or the peptide preparation described above may be used in the preparation of a hydrogel for medical uses.

In a fifth aspect, the present invention provides an application of The self-assembling peptide, the peptide composition or the peptide preparation described above may be used in culturing cells.

The self-assembling peptide, the peptide composition or the peptide preparation described above may be used in in the preparation of a cell culture scaffold.

The beneficial effects of the present invention include the following:
1. The self-assembling peptide provided by the present invention can remain in solution state at a concentration of 1% w/v under the condition of pH<6.0.
2. A 2.5% w/v peptide solution of the self-assembling peptide provided by the present invention, when mixed with DMEM at a volume ratio of 1:1, can reach a pH value of around 6, indicating that the peptide solution is more easily neutralized by the cell culture medium.
3. A peptide hydrogel formed by the self-assembling peptide provided by the present invention can maintain good cell proliferation, exhibits lower cytotoxicity and good cytocompatibility.
4. The self-assembling peptide provided by the present invention has good liquid-solid phase transition ability and better mechanical performance.

### BRIEF DESCRIPTION OF THE DRAWINGS

The drawings accompanying the specification forming a part of the present invention serve to provide a further understanding of the present invention. The schematic embodiments of the present invention and their description are used to explain the present invention and do not constitute an improper limitation of the present invention.
FIG. 1 shows a high-performance liquid chromatography (HPLC) profile of PRRVDP13 prepared in one example of the present invention.
FIG. 2 shows a mass spectrum of PRRVDP13 prepared in one example of the present invention.
FIG. 3 shows a pH value result of an experiment in which the peptide solution and DMEM cell culture medium were mixed in certain volume ratios in one example of the present invention (n=6, ***P<0.001).
FIG. 4 shows images of the viability of cells cultured on surfaces of different peptides in one example of the present invention. A shows the RADA16 group, B shows the PRVDP9 group, C shows the PRRVDP13 group, and D shows the control group (culture plate without peptide).
FIG. 5 presents a bar chart of the results of the cytotoxicity assay in one example of the present invention (n=5, ***p < 0.001).

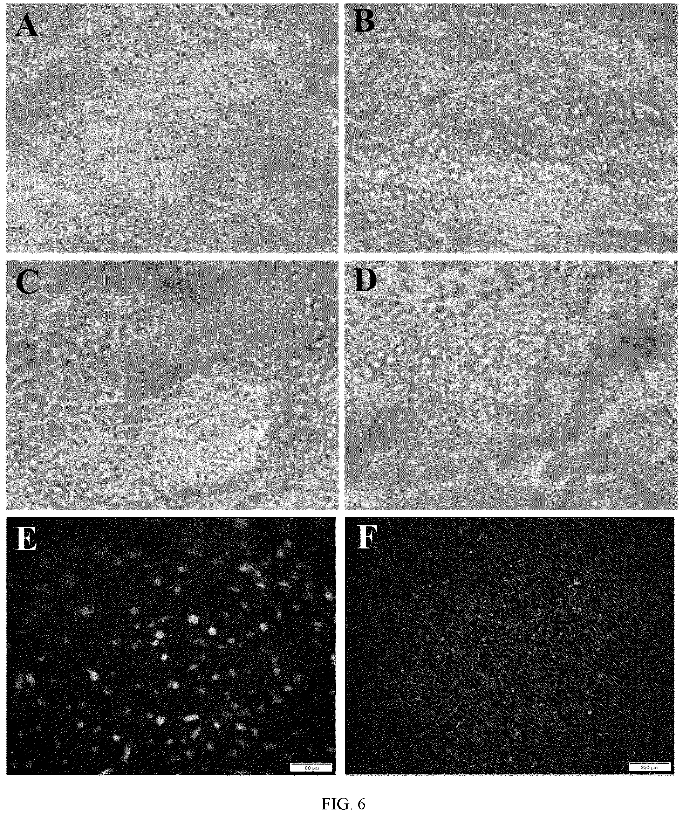

FIG. 6 shows images of PRRVDP13 peptide solutions mixed in equal volumes with DMEM culture medium in one example of the present invention. A shows the cell image with cell suspension added to 2.5% w/v PRRVDP13 peptide solution; B shows the cell image with 2.5% w/v PRRVDP13 peptide solution added to cell suspension; C shows the cell image with cell suspension added to 1.25% w/v PRRVDP13 peptide solution; D shows the cell image with 1.25% w/v PRRVDP13 peptide solution added to cell suspension; E shows the fluorescence stained image (Calcein-AM and PI, Beyotime, C2015S) with cell suspension added to 2.5% w/v PRRVDP13 peptide solution under 10 × objective (scale bar = 100 µm); F is a fluorescence stained image (Calcein-AM and PI, Beyotime, C2015S) with cell suspension added to 2.5% w/v PRRVDP13 peptide solution under 5 × objective (scale bar = 200 µm).
FIG. 7 shows mechanical property characterization of peptides in one example of the present invention. A shows the storage modulus and loss modulus of RADA16; B shows the storage modulus and loss modulus of PRVDP9; C shows the storage modulus and loss modulus of PRRVDP13; D shows the shear viscosity of RADA16; E shows the shear viscosity of PRVDP9; and F shows the shear viscosity of PRRVDP13.
FIG. 8 shows images of the liquid (left) and solid (right) states of the PRRVDP13 peptide in one example of the present invention.

### DETAILED DESCRIPTION

It should be noted that the following detailed descriptions are provided as examples to further illustrate the present invention. Unless otherwise indicated, all technical and scientific terms used in this document have the same meanings as commonly understood by those skilled in the art to which the invention pertains.

It should be understood that the terms used here are for the purpose of describing specific embodiments and are not intended to limit the present invention as defined by the exemplary embodiments. As used here, unless otherwise specified in the context, the singular form also includes the plural form. Additionally, it should be understood that when the terms "comprises" and/or "comprising" are used in this specification, they specify the presence of features, steps, operations, devices, components, and/or combinations thereof.

Given the problem of low acidity and difficulty in neutralization of existing self-assembling peptides, which hinders their application in cell culture and *in vivo* use, the present invention proposes a self-assembling peptide whose amino acid sequence consists of the amino acid sequence set forth in SEQ ID NO: 1.

In some embodiments, the N-terminus (i.e., the amino terminus of the self-assembling peptide) is modified to an acetylamino group (CH₃CONH-).

In some embodiments, the C-terminus (i.e., the carboxy terminus of the self-assembling peptide) is modified to an amide group (-CONH₂).

In some embodiments, a peptide composition comprises a self-assembling peptide as described above and at least a pharmaceutical excipient.

Suitable pharmaceutical excipients include, for example, pure water, alkaline solution containing NaHCO₃ or NaOH, buffer solution, osmolarity-adjusted solution containing substance such as sucrose or mannitol, and cell culture medium.

In some embodiments, a peptide preparation comprises a self-assembling peptide as described above, and the peptide preparation is in a form of a powder or a solution.

When the peptide preparation is a powder, the powder is used by dissolving it in a solution such as pure water, alkaline solution containing NaHCO₃ or NaOH, buffer solution, or cell culture medium.

In some embodiments, the peptide preparation is a solution, wherein a concentration of the self-assembling peptide is from 0.1% to 5% w/v, preferably is from 0.1% to 1.25% w/v, and preferably is from 1.25% to 5% w/v.

In some embodiments, the peptide preparation is a solution, wherein a concentration of the self-assembling peptide is 1% w/v, and a pH value is less than 6.29, preferably is 2.50-3.50, and preferably is 5.50-6.28.

The self-assembling peptide, the peptide composition or the peptide preparation as described above may be used in the preparation of a hydrogel for medical uses. In particular, the hydrogel for medical uses is used for hemostasis and/or sealing of body fluids.

The self-assembling peptide, the peptide composition or the peptide preparation as described above may be used in culturing cells.

The self-assembling peptide, the peptide composition or the peptide preparation as described above may be used in the preparation of a cell culture scaffold.

For cell culture, a cell suspension may be mixed with the self-assembling peptide as described above to form a gel, and a culture medium may then be added for culturing.

In order for those skilled in the art to better illustrate the technical solution of the present invention, the following will further describe the present invention with reference to specific embodiments.

### Examples

Synthesis of PRRVDP13 (AC-PRVDVRVRVRVDP-NH₂, as shown in SEQ ID NO: 1)

### 1. Materials

Fmoc-Pro-OH (N-fluorenylmethyloxycarbonyl-proline), Fmoc-Val-OH (N-fluorenylmethyloxycarbonyl-valine), Fmoc-Arg (pbf)-OH (N-fluorenylmethyloxycarbonyl-2, 2, 4, 6, 7-pentamethyldihydrobenzofuran-5-sulfonyl-arginine), Fmoc-Asp (OtBu)-OH (N-fluorenylmethyloxycarbonyl-4 tert-butyl ester-aspartic acid) were purchased from GL Biochem (Shanghai) Ltd., Rink Amide-AM Resin (Resin) was purchased from Sunresin New Materials (Xi'an) Co., Ltd., DIC (N, N'-Diisopropylcarbodiimide), HOBT (1-Hydroxybenzotriazole), DIEA (N, N-Diisopropylethylamine) were purchased from Suzhou Highfine Biotech Co., Ltd. PIPE (piperidine), acetic anhydride, DMF (N, N-dimethylformamide), TFA (trifluoroacetic acid), methyl tert-butyl ether, methanol and DCM (dichloromethane) were purchased from Sinopharm Chemical Reagent Ltd.

### 2. Synthetic Methods

The Fmoc solid-phase synthesis method was used. Firstly, 2.10 g of Rink Amide-AM resin was weighed and soaked in 20 mL of DCM for one hour. The DCM was then removed by vacuum filtration, and the resin was washed twice with DMF. Next, 20 mL of a 20% v/v PIPE/DMF solution was added to remove the Fmoc protecting group, and after 10 minutes, the resin was filtered under reduced pressure, this process was repeated once. The resin was then washed six times with DMF. The removal of the protecting group was checked using ninhydrin.

Then, 3.04 g of Fmoc-Pro-OH and 1.34 g of HOBT were weighed and dissolved in 15 mL of DMF. 1.25g of DIC was added for activation, and then the mixture was added to the reactor and reacted for 2 hours. A small amount of resin was taken to check the completeness of the coupling. After complete coupling, the resin was filtered under reduced pressure and washed with DMF six times. Then, a 20% v/v PIPE/DMF solution was added to remove the Fmoc protecting group of the amino acid.

The same steps were repeated for each amino acid from the C-terminal to the N-terminal. Once all the amino acids were connected, the protecting group of the last amino acid was removed, and the resin was washed. Then, 5.7 mL of acetic anhydride, 10.5 mL of DIEA, and 10 mL of DCM were added to the resin and reacted for 30 minutes for acetylation of the N-terminus. After the reaction, the resin was washed repeatedly with DMF, DCM, and methanol, and then vacuum-dried overnight.

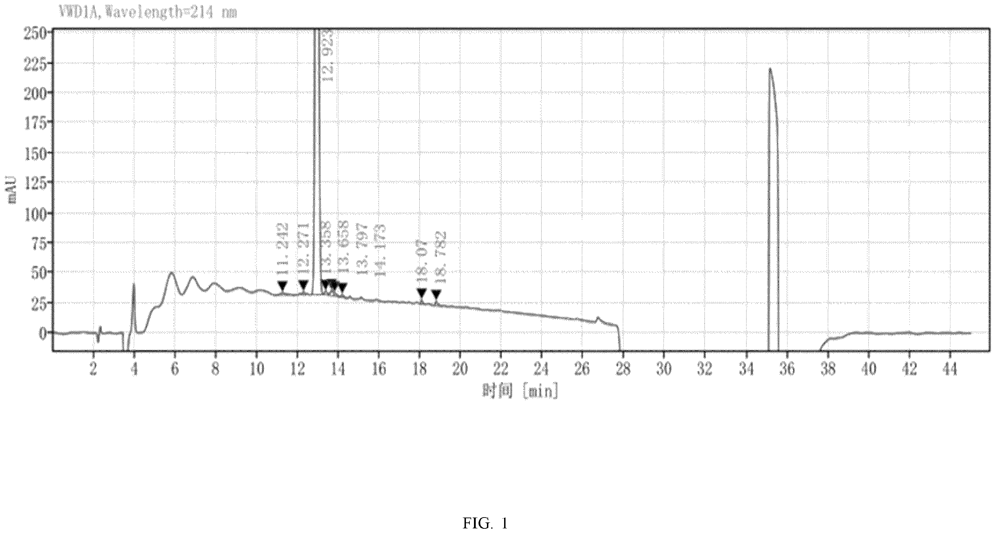

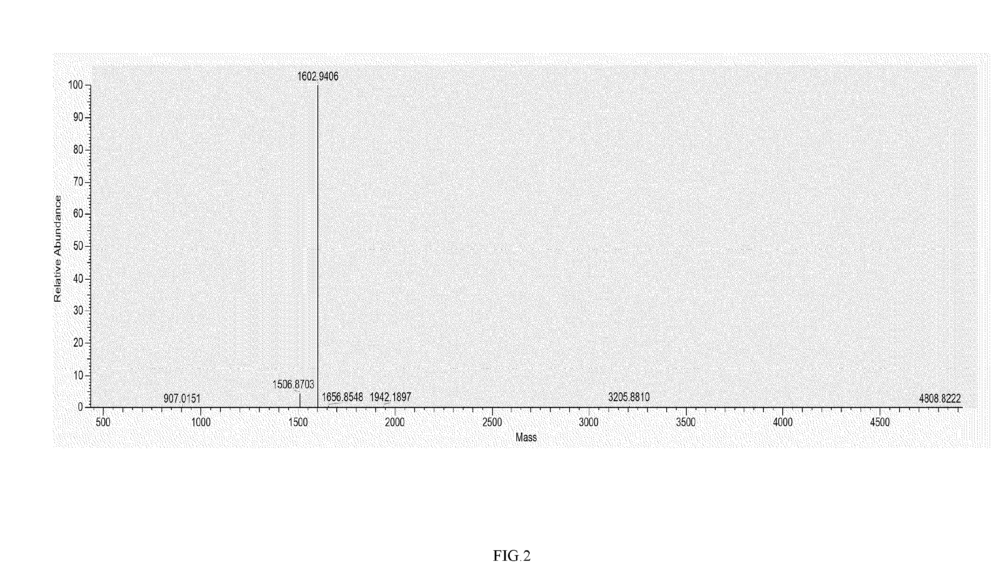

The dried resin was added to 30 mL of pre-cooled cleaving reagent and stirred at room temperature for 2.5 hours. After the reaction, the mixture was filtered under reduced pressure, and the filtrate was added slowly and dropwisely to pre-cooled methyl tert-butyl ether, resulting in a white precipitate. The precipitate was collected by centrifugation and vacuum-dried, yielding crude peptide powder. The crude peptide powder was purified using a high-performance liquid chromatography (HPLC), and the target peak was collected. Alternatively, the crude peptide powder was converted to acetate or hydrochloride salt using HPLC, and the target peak was collected. Finally, the pure product, namely PRRVDP13, was obtained by concentration and freeze-drying, with a purity of up to 97%, as shown in FIGS. 1-2.

According to the above synthesis method, by changing the amino acids and their ratios, the following peptides are synthesized:
AC-RADARADARADARADA-NH₂ (RADA16, as shown in SEQ ID NO: 2);
AC-PRVDVRVDP-NH₂ (PRVDP9, as shown in SEQ ID NO: 3);
AC-KLNLKLNLNL-NH₂ (as shown in SEQ ID NO: 4);
AC-PRVDVRVKVRVDP-NH₂ (as shown in SEQ ID NO: 5);
AC-RADMSRADMS-NH₂ (as shown in SEQ ID NO: 6).

Unless otherwise specified, the solutions in this example refer to aqueous solutions prepared using purified water or sterile water as the solvent. Based on the AC-PRVDVRVDP-NH₂ provided previously, the peptides AC-KLNLKLNLNL-NH₂, AC-PRVDVRVKVRVDP-NH₂, and AC-RADMSRADMS-NH₂ were screened. The above-mentioned peptides were formulated into a 1 mL 2.5% w/v solution, to which 100 µL of 10-fold concentration PBS buffer was added and thoroughly mixed to achieve a final solution of approximately 1-fold PBS. Alternatively, an equal volume of DMEM was added for gel formation, and it was found that only AC-PRVDVRVKVRVDP-NH₂ dissolved easily and formed a good gel when mixed with DMEM or PBS. Based on this, a similar sequence with liquid-solid phase transition capability, AC-PRVDVRVRVRVDP-NH₂ (PRRVDP13), was further designed. At a concentration of 1% w/v, the pH of the two peptide solutions could be pre-adjusted to a certain level using a dropwise addition of 1% w/v NaHCO₃ and shaking, without the peptide solution turning into a gel for several hours. However, at a concentration of 2% w/v, when the pH increased to around 3 using a dropwise addition of 1% w/v NaHCO₃ and shaking, the peptide solution quickly turned into a gel. The classical sequence RADA16 and the original sequence PRVDP9 needed to be maintained under acidic conditions even at a lower concentration of 1% w/v to keep them in solution state. Otherwise, the solution would turn into a gel, and the color would turn white as the pH increased. The experimental results are shown in Table 1.

**Table 1**

| Peptide Sequences | Solution Concentration (w/v) | State of Peptide Under Different pH Conditions | |
|---|---|---|---|
| AC-PRVDVRVRVRVDP-NH₂ (PRRVDP13) | 1% | pH < 6.29, solution | pH ≥ 6.29, gel |
| | 2% | pH < 3.25, solution | pH ≥ 3.25, gel |
| AC-PRVDVRVKVRVDP-NH₂ | 1% | pH < 4.05, solution | pH ≥ 4.05, gel |
| | 2% | pH < 3.20, solution | pH ≥ 3.20, gel |
| AC-PRVDVRVDP-NH₂ (PRVDP9) | 1% | pH < 3.50, solution | pH ≥ 3.50, gel |
| RADA16 | 1% | pH < 3.40, solution | pH ≥ 3.40, gel |

One potential application of ISAPs is to use them as scaffolds for supporting 3D cell culture. To validate their suitability as a 3D cell culture scaffold, L929 cells (purchased from the Kunming Cell Bank of the Chinese Academy of Sciences) that had been cultured for 48-72 hours and were in a vigorous growth phase were digested and then mixed with serum-containing DMEM medium for cell counting. The cell suspension was prepared at a concentration of 1×10⁵ cells/mL with serum-containing DMEM medium. The cell suspension was mixed with the peptide in a specific ratio (60 µL:60 µL) and added to a 96-well culture plate. After gelation, 120 µL of serum-containing DMEM medium was added for cell culture. In this example, the ISAP solution was mixed with the cell suspension or cell culture medium to study their biocompatibility.

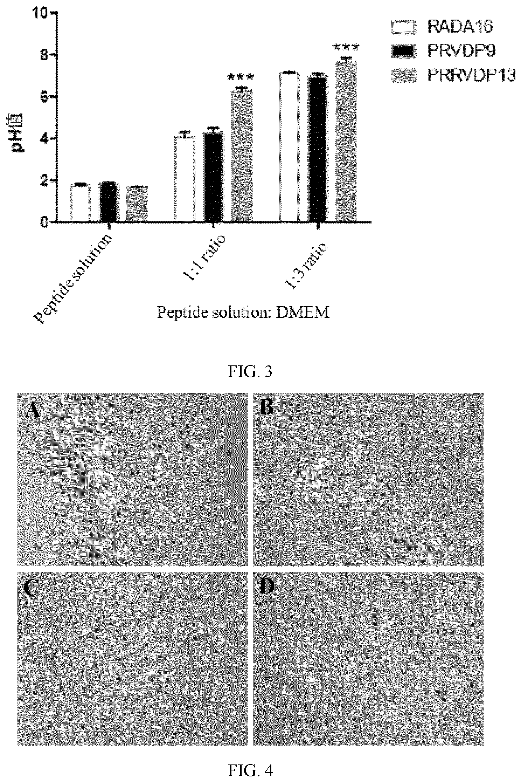

As mentioned above, peptide solutions with relatively high concentrations (over 2% w/v) cannot be pre-adjusted to neutral pH before mixing with the cell suspension, and the acidity may be harmful to the cells. However, surprisingly, the acidity of the 2.5% w/v peptide solution of PRRVDP13 was easily neutralized by mixing with DMEM medium, while the control solutions of PRVDP9 and RADA16 were relatively more difficult to neutralize. The results, as shown in FIG. 3, indicate that at the same volume ratio, the pH of the PRRVDP13-DMEM mixture was significantly higher than that of the PRVDP9 and RADA16 at a concentration of 2.5% w/v, with statistical significance. Therefore, despite the acidic nature of the 2.5% concentration solution, PRRVDP13 may still be used as a cell culture scaffold. The experimental methods are as follows. In a centrifuge tube, 1.5 mL of a 2.5% w/v self-assembling peptide solution was mixed with 1.5 mL of DMEM medium, and after gelation, additional medium was added to make a total volume of 6 mL, with a final peptide-to-medium ratio of 1:3. Alternatively, 1.5 mL of a 2.5% self-assembling peptide solution was mixed with 1.5 mL of DMEM medium to achieve a peptide-to-DMEM medium ratio of 1:1. The pH value of each group was then measured.

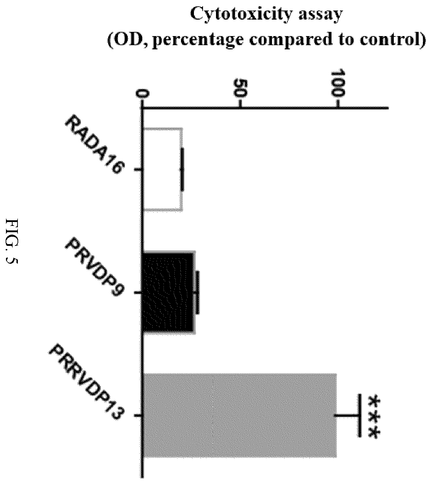

To test the biocompatibility of ISAPs, 60 µL of a 2.5% w/v peptide solution was mixed with an equal volume of DMEM medium in a cell culture plate to form a gel. Then, 120 µL of the aforementioned 1×10⁵ cells/mL L929 cell suspension was loaded onto the pre-formed gel. The cells showed good proliferation on the PRRVDP13 peptide gel, but not on the gels of other sequences, as shown in FIGs. 4 and 5. These results indicate that PRRVDP13 has excellent cell compatibility, is easy to handle, and can be used at relatively high concentrations. The cell proliferation assay was performed after culturing the cells in a cell incubator at an atmosphere of 5% CO₂ for approximately 48 hours using a CCK-8 assay kit (Beyotime, C0038). For the PRRVDP13 peptide, the cell suspension was directly mixed with the peptide solution, and after the liquid-to-solid phase transition, cells were evenly distributed within the gel. The methods are as follows. 60 µL of the aforementioned L929 cell suspension of 1×10⁵ cells/mL and 60 µL of PRRVDP13 peptide were added to a 96-well culture plate. After gelation, 120 µL of culture medium was added for cell culture. After approximately 48 hours of incubation in a cell incubator at an atmosphere of 5% CO₂, cells were observed directly using a microscope or observed using a fluorescence microscope following staining with Calcein-AM and PI (Beyotime, C2015S) according to the manufacturer's instructions. The results showed that the majority of cells were viable, as shown in FIG. 6.

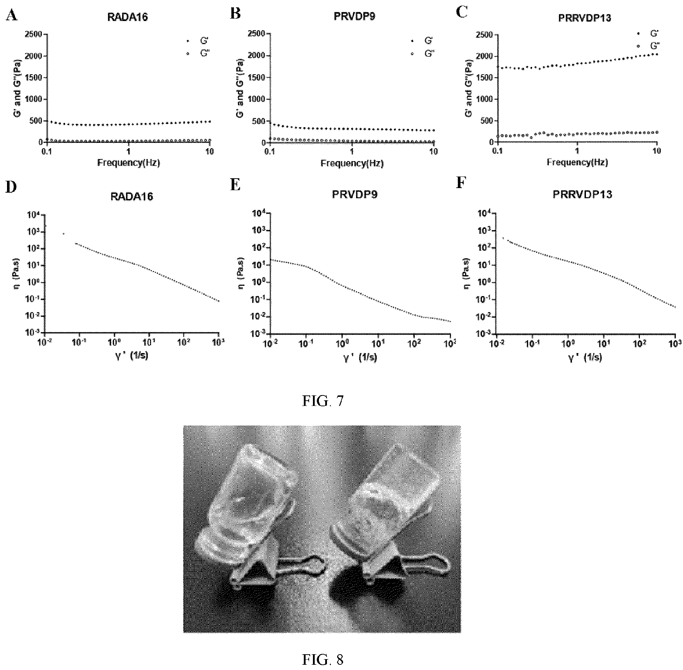

As shown in FIG. 8, the PRRVDP13 peptide solution exhibited good liquid-to-solid phase transition ability. The experimental methods are as follows. Peptide was prepared as a 1 mL solution with a concentration of 2.5% w/v. 100 µL of a 10-fold concentration PBS buffer was added and thoroughly mixed to make the final solution as approximately 1-fold PBS to trigger gelation. The gel strength, which is related to hemostatic ability, is usually represented by the storage modulus G'. Compared to RADA16 and PRVDP9, PRRVDP13 has better mechanical properties, as shown in FIGs. 7A-7C. The experimental methods are as follows. 3 mL of a 2.5% w/v solution of each peptide was prepared using water for injection and then 600 µL of a 5-fold concentration PBS buffer was added in to trigger gel formation. A suitable amount of gel was placed on the rheometer testing stage, with the setting of the stress of the rheometer at 1 Pa, and the scanning frequency range from 0.1 to 10 Hz to measure the storage modulus G' and loss modulus G" of the gel. To measure the viscosity of the peptide solutions, each peptide was prepared into a 2.5% w/v solution using water for injection, and a creep scan was performed using a rheometer at shear rates ranging from 0.001S⁻¹ to 1000S⁻¹ to obtain viscosity data for each peptide solution, as shown in FIGs. 7D-7F.

The PRRVDP13 peptide solution showed better hemostatic effects. The animal (New Zealand rabbits) was anesthetized by 2% sodium pentobarbital via ear vein injection. The animal was then fixed in a supine position on the operating table. The abdominal fur was shaved, and the area was sterilized. A standard midline incision was made, and the left lobe of liver was exposed by dissecting the liver. A sterile gauze was placed beneath the liver. A 1.0 cm long and 0.3 cm deep wound was created on the liver lobe, and the wound was wiped with gauze. The test sample (approximately 1 mL) was injected deeply onto the wound until the entire wound area was covered. The hemostatic effect was observed and the hemostasis time was recorded. The results showed that a 2.5% w/v self-assembling peptide solution of PRRVDP13, or a 1.25% w/v self-assembling peptide (solution with a concentration of 1 mg/mL NaHCO₃), achieved complete hemostasis within 50 seconds and formed a solid gel. After 2 minutes of hemostasis, peptide hydrogel on the surface was rinsed off with normal saline, and no new bleeding appeared. The control group, treated with normal saline, had a bleeding time of over 4 minutes.

## Claims

1. A self-assembling peptide whose amino acid sequence consists of the amino acid sequence set forth in SEQ ID NO: 1.

2. The self-assembling peptide according to claim 1, wherein an N-terminus of the self-assembling peptide is modified to an acetylamino group.

3. The self-assembling peptide according to claim 1, wherein a C-terminus of the self-assembling peptide is modified to an amide group.

4. A peptide composition comprising the self-assembling peptide according to any one of claims 1 to 3 and at least a pharmaceutical excipient.

5. A peptide preparation comprising the self-assembling peptide according to any one of claims 1 to 3, and the peptide preparation is in a form of a powder or a solution.

6. The peptide preparation according to claim 5, wherein the peptide preparation is the solution, wherein a concentration of the self-assembling peptide is from 0.1% to 5% w/v.

7. The peptide preparation according to claim 5, wherein the peptide preparation is the solution, wherein a concentration of the self-assembling peptide is 1% w/v, and a pH value is less than 6.29.

8. An application of the self-assembling peptide according to any one of claims 1 to 3, the peptide composition according to claim 4 or the peptide preparation according to any one of claims 5 to 7 in the preparation of a hydrogel for medical use.

9. An application of the self-assembling peptide according to any one of claims 1 to 3, the peptide composition according to claim 4 or the peptide preparation according to any one of claims 5 to 7 in culturing cells.

10. An application of the self-assembling peptide according to any one of claims 1 to 3, the peptide composition according to claim 4 or the peptide preparation according to any one of claims 5 to 7 in the preparation of a cell culture scaffold.

## Patentansprüche

1. Selbstassemblierendes Peptid, dessen Aminosäuresequenz aus der Aminosäuresequenz, die in SEQ ID NO: 1 vorgegeben ist, besteht.

2. Selbstassemblierendes Peptid nach Anspruch 1, wobei ein N-Terminus des selbstassemblierenden Peptids zu einer Acetylaminogruppe modifiziert ist.

3. Selbstassemblierendes Peptid nach Anspruch 1, wobei ein C-Terminus des selbstassemblierenden Peptids zu einer Amidgruppe modifiziert ist.

4. Peptidzusammensetzung, umfassend das selbstassemblierende Peptid nach einem der Ansprüche 1 bis 3 und mindestens einen pharmazeutischen Hilfsstoff.

5. Peptidzubereitung, umfassend das selbstassemblierende Peptid nach einem der Ansprüche 1 bis 3, wobei die Peptidzubereitung in Form eines Pulvers oder einer Lösung vorliegt.

6. Peptidzubereitung nach Anspruch 5, wobei die Peptidzubereitung die Lösung ist, wobei eine Konzentration des selbstassemblierenden Peptids 0,1 % bis 5 % m/V beträgt.

7. Peptidzubereitung nach Anspruch 5, wobei die Peptidzubereitung die Lösung ist, wobei eine Konzentration des selbstassemblierenden Peptids 1 % m/V beträgt und ein pH-Wert geringer als 6,29 ist.

8. Anwendung des selbstassemblierenden Peptids nach einem der Ansprüche 1 bis 3, der Peptidzusammensetzung nach Anspruch 4 oder der Peptidzubereitung nach einem der Ansprüche 5 bis 7 bei der Herstellung eines Hydrogels zur medizinischen Verwendung.

9. Anwendung des selbstassemblierenden Peptids nach einem der Ansprüche 1 bis 3, der Peptidzusammensetzung nach Anspruch 4 oder der Peptidzubereitung nach einem der Ansprüche 5 bis 7 beim Kultivieren von Zellen.

10. Anwendung des selbstassemblierenden Peptids nach einem der Ansprüche 1 bis 3, der Peptidzusammensetzung nach Anspruch 4 oder der Peptidzubereitung nach einem der Ansprüche 5 bis 7 bei der Herstellung eines Zellkulturgerüsts.

## Revendications

1. Peptide à auto-assemblage dont la séquence d'acides aminés est constituée par la séquence d'acides aminés représentée dans SEQ ID NO: 1.

2. Peptide à auto-assemblage selon la revendication 1, dans laquelle une extrémité N-terminale de la peptide à auto-assemblage est modifiée en un groupement acétylamino.

3. Peptide à auto-assemblage selon la revendication 1, dans laquelle une extrémité C-terminale de la peptide à auto-assemblage est modifiée en un groupement amide.

4. Composition peptidique comprenant la peptide à auto-assemblage selon l'une quelconque des revendications 1 à 3 et au moins un excipient pharmaceutique.

5. Préparation peptidique comprenant la peptide à auto-assemblage selon l'une quelconque des revendications 1 à 3, et la préparation peptidique est sous la forme d'une poudre ou d'une solution.

6. Préparation peptidique selon la revendication 5, dans laquelle la préparation peptidique est la solution, dans laquelle une concentration de la peptide à auto-assemblage est de 0,1 % à 5 % p/v.

7. Préparation peptidique selon la revendication 5, dans laquelle la préparation peptidique est la solution, dans laquelle une concentration de la peptide à auto-assemblage est de 1 % p/v, et une valeur de pH est inférieure à 6,29.

8. Application de la peptide à auto-assemblage selon l'une quelconque des revendications 1 à 3, de la composition peptidique selon la revendication 4 ou de la préparation peptidique selon l'une quelconque des revendications 5 à 7 dans la préparation d'un hydrogel pour utilisation médicale.

9. Application de la peptide à auto-assemblage selon l'une quelconque des revendications 1 à 3, de la composition peptidique selon la revendication 4 ou de la préparation peptidique selon l'une quelconque des revendications 5 à 7 dans la culture cellulaire.

10. Application de la peptide à auto-assemblage selon l'une quelconque des revendications 1 à 3, de la composition peptidique selon la revendication 4 ou de la préparation peptidique selon l'une quelconque des revendications 5 à 7 dans la préparation d'un échafaudage de culture cellulaire.
